# NOUVEAU FASCICULE DE BREVET EUROPEEN

(11) **EP 0 553 241 B2**
(45) Date de publication et mention de la décision concernant l'opposition: **12.09.2001**
(45) Mention de la délivrance du brevet: 12.04.1995
(21) Numéro de dépôt: 91919574.3
(22) Date de dépôt: 16.10.1991
(51) Int. Cl.: B01F 17/56, B01F 17/38, A61K 7/00

(54) **UTILISATION DE COMPOSITIONS A BASE D'ALCOOLS GRAS, POUR LA PREPARATION D'EMULSIONS; PROCEDE DE PREPARATION D'EMULSIONS ET EMULSIONS AINSI OBTENUES**
VERWENDUNG VON GEMISCHEN AUF DER BASIS VON FETTALKOHOLEN ZUR HERSTELLUNG VON EMULSIONEN, VERFAHREN ZUR HERSTELLUNG VON EMULSIONEN UND DANACH HERGESTELLTE EMULSIONEN
USE OF FATTY ALCOHOL BASED COMPOSITIONS FOR PREPARING EMULSIONS, METHOD OF PREPARING EMULSIONS AND EMULSIONS SO OBTAINED

(30) Priorité: 17.10.1990 FR 9012842
(43) Date de publication de la demande: 04.08.1993
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: BRANCQ, Bernard, F-78150 Le Chesnay (FR); BOITEUX, Jean-Pierre, F-81710 Saix (FR)
(74) Mandataire: Hubert, Philippe
(86) Numéro de dépôt international: FR9100804
(87) Numéro de publication internationale: WO9206778

(56) Documents cités:
- EP-A- 77 167
- EP-A- 230 598
- EP-A- 336 000
- EP-A- 345 586
- JP-A- 89 203 036
- US-A- 3 839 318

## Description

La présente invention concerne généralement l'utilisation de compositions à base d'alcools gras, pour la préparation d'émulsions et un procédé de préparation d'émulsions.

L'invention trouve notamment application dans les domaines cosmétiques et pharmaceutiques.

On sait que les émulsions généralement utilisées dans les domaines cosmétiques et pharmaceutiques comprennent essentiellement des émulsionnants non ioniques, du type ester ou éther de polyol (glycérol, sorbitol, glycol, polyglycérol, etc..), dont la fonction est de rendre compatibles les phases grasses lipophiles et la phase aqueuse.

On sait également que certaines compositions à base d'alcool, acide ou ester gras, peuvent être rendues "auto-émulsionnables" en utilisant, comme émulsionnant, un produit tensioactif de nature non ionique, de type hydrophile, obtenu par greffage d'une chaîne polyoxyéthylée.

On entend désigner ici par l'expression "auto-émulsionnable" une composition permettant d'obtenir très facilement des émulsions stables par simple dispersion à chaud par agitation, notamment mécanique, lente, dans de l'eau ou un milieu polaire approprié.

Des exemples de telles compositions auto-émulsionnables sont le CETOMACROGOL EMULSIFYING WAX décrit dans la British Pharmacopea ou encore les produits commercialisés sous les dénominations CIRE DE LANOL CTO (Société SEPPIC), SINNOWAX AO (Société HENKEL) ou encore, PROMULGEN G (Société AMERCHOL).

Ces compositions sont généralement constituées par :
- 60 à 90% en poids d'alcools gras lourds (C₁₆-C₁₈);
- 10 à 40% en poids d'alcool cétylstéarylique condensé avec 20 à 35 molécules d'oxyde d'éthylène.

On connaît également des cires à base d'ester gras rendues auto-émulsionnables par addition d'acide gras polyoxyéthylé. Un exemple de ce type de produit est l'ARLACEL 165 (Société ICI) constitué par environ 50 % en poids de stéarate de glycérol et 50 % en poids d'acide stéarique condensé avec 100 molécules d'oxyde d'éthylène.

De telles compositions auto-émulsionnables connues permettent d'obtenir très facilement des émulsions très stables et pouvant contenir des molécules actives dispersées ou dissoutes (analgésiques, antibiotiques, antiseptiques, hydratantes, anti-ultraviolets, conditionneurs capillaires, etc.) par exemple par dispersion à chaud (50 à 80°C) dans de l'eau ou des milieux polaires appropriés, par simple agitation mécanique lente.

Cependant, toutes ces compositions auto-émulsionnables sont obtenues par la mise en oeuvre d'un procédé comportant une étape de condensation d'oxyde d'éthylène, et peuvent donc présenter des impuretés liées à ce procédé, comme par exemple le dioxane 1-4 ou l'oxyde d'éthylène qui sont généralement considérés comme des produits toxiques et préjudiciables à la santé.

Dans ces conditions, la présente invention a essentiellement pour but de résoudre le problème technique consistant en la fourniture de nouvelles compositions auto-émulsionnables à base d'alcool gras n'ayant pas les inconvénients précités.

Il a été découvert, et ceci constitue le fondement de la présente invention, que des compositions à base d'alcools gras comprenant :
- 60 à 90 % en poids d'un alcool gras, ayant de 12 à 22 atomes de carbone ;
- 10 à 40 % en poids d'un alkylpolyoside, de préférence un alkylpolyoside dont la partie alkyle est identique à celle de l'alcool gras ;
- et éventuellement 0,5 à 5 % en poids de polyosides présentent la propriété inattendue d'être auto-émulsionnables et que de telles compositions peuvent avantageusement être utilisées pour la préparation d'émulsions.

Ainsi, par simple dispersion à chaud dans l'eau ou un polyol hydrophile, ces compositions conduisent à des émulsions stables, sans avoir recours à la technologique classique des émulsions où l'on met en oeuvre un couple d'émulsionnants (l'un hydrophile, l'autre lipophile).

De telles compositions auto-émulsionnables peuvent être obtenues par des procédés qui, contrairement à ceux utilisés jusqu'à présent, ne génèrent aucune impureté toxique.

Il est à noter que les alkylpolyosides sont des surfactants non ioniques déjà utilisés dans une large gamme d'applications industrielles.

Des compositions émulsifiantes comprenant de l'octadécanol et, en majeure partie, les alkylpolyosides correspondants, sont décrits dans US-A-3.839.318 (cf. Table I, exemple 8, N°. S et T).

Cependant, ces composés n'ont jamais été utilisés comme émulsionnants dans la préparation de compositions auto-émulsionnables.

Des compositions comprenant un alkylpolyoside et un alcool gras sont également implicitement révélées par le document EP 0.077.167, mais il est à noter que ces compositions constituent seulement des compositions intermédiaires non isolées; l'alcool en excès étant éliminé.

Ce sont les compositions finales constituées exclusivement d'alkylpolyoside qui trouvent utilisation.

Ainsi, selon un premier aspect, l'invention vise à couvrir l'utilisation de compositions à base d'alcools gras comprenant :
- 60 à 90 % en poids d'au moins un alcool gras ayant de 12 à 22 atomes de carbone, de préférence de 16 à 18 atomes de carbone,
- 10 à 40 % en poids d'un alkylpolyoside, de préférence un alkylpolyoside dont la partie alkyle est identique à celle de l'alcool gras,
- et éventuellement 0,5 à 5% de polyoside comme compositions auto-émulsionnables pour la préparation d'émulsions.

Selon un second aspect, l'invention vise à couvrir un procédé de préparation d'émulsions, caractérisé en ce qu'il comprend :
a) la préparation d'une composition auto-émulsionnable comprenant :
   - 60 à 90 % en poids d'au moins un alcool gras ayant de 12 à 22 atomes de carbone, de préférence de 16 à 18 atomes de carbone,
   - 10 à 40 % en poids d'un alkylpolyoside, de préférence un alkylpolyoside dont la partie alkyle est identique à celle de l'alcool gras,
   - et éventuellement 0,5 à 5% de polyoside ; et
b) la dispersion à chaud de ladite composition, par exemple entre environ 50 °C et environ 80 °C, dans de l'eau ou un milieu polaire approprié, par simple agitation, notamment mécanique, lente.

Il est à noter qu'il existe, à l'échelle industrielle, deux voies principales de synthèse des alkylpolyosides.

La première voie comprend la réaction, en milieu acide, entre un alcool et un ose disposant d'un OH anomérique, comme par exemple le glucose ou le dextrose.

Lorsque l'on fait réagir un alcool gras lourd, ayant par exemple de 8 à 22 atomes de carbone, la réaction est incomplète et nécessite une distillation de l'alcool gras en excès.

La deuxième voie consiste :
- dans une première étape, à préparer un alkylpolyoside avec un alcool léger comme par exemple le méthanol ou le butanol par éthérification d'un ose, comme par exemple le glucose, pour obtenir des produits connus tels que méthylglucoside ou butylglycoside avec un rendement élevé ; puis
- dans une seconde étape, à effectuer une transéthérification avec un alcool gras lourd, ayant par exemple de 8 à 22 atomes de carbone, avec distillation de l'alcool léger (méthanol ou butanol).

Il a été découvert, de façon tout à fait surprenante, que les mélanges d'alkylpolyoside et d'alcools gras lourds en excès, obtenus par la première voie classique de synthèse précitée (sans distillation), présentaient des propriétés auto-émulsionnables particulièrement intéressantes.

C'est pourquoi, les compositions auto-émulsionnables sont de préférence obtenues directement par réaction, en milieu acide, entre un alcool gras ayant de 12 à 22 atomes de carbone et un ose, suivie éventuellement d'une neutralisation et d'une filtration.

Comme on le comprend, l'alcool gras est utilisé en excès, de telle sorte que le produit de la réaction contienne les quantités précédemment spécifiées d'alcools gras non éthérifiés et d'alkylpolyoside.

Il a également été constaté que l'on pouvait également obtenir des compositions auto-émulsionnables par simple mélange, dans les proportions indiquées précédemment, d'au moins un alcool gras ayant de 12 à 22 atomes de carbone, de préférence de 16 à 18 atomes de carbone, et d'un alkylpolyoside, dont la partie alkyle est de préférence identique à celle de l'alcool gras ; cet alkylpolyoside pouvant être obtenu par toute méthode connue et notamment par les deux voies principales de synthèse mentionnées ci-dessus.

Avantageusement, l'alkylpolyoside précité comprend au moins un ose choisi parmi le groupe constitué de glucose ou dextrose, saccharose, fructose, galactose, maltose, maltotriose, lactose, cellobiose, mannose, ribose, dextrane, talose, allose, xylose, levoglucosane, cellulose et amidon ; de préférence glucose, dextrose, fructose et maltose.

Généralement, la chaîne polyoside peut comprendre jusqu'à 30 unités.

Il est en outre à noter que chaque unité de la partie polyoside peut être sous forme isomérique α ou β, sous forme L ou D, et la conformation du motif "ose" sous forme furanoside ou pyranoside avec un oxygène anomérique.

Les alkylpolyosides visés par la présente invention sont généralement les éther-alkyles de polysaccharide (ou oligosaccharide).

Par ailleurs, les alcools gras pouvant être utilisés pour la réalisation de compositions auto-émulsionnables selon l'invention sont généralement des alcools linéraires ou ramifiés, d'origine naturelle, comme par exemple les alcools provenant de matières végétales (coprah, palmiste, palme) ou animales (suif) ; ou encore synthétiques, et dont la longueur de chaîne est comprise entre 12 et 22 atomes de carbone, de préférence entre 14 et 22 atomes de carbone.

Bien entendu, d'autres alcools à longue chaîne peuvent également être utilisés, comme par exemple les étheralcools ou bien encore les alcools dits de Guerbet.

Enfin, on peut également utiliser certaines coupes plus ou moins longues d'alcool d'origine naturelle, comme par exemple coco (C₁₂ à C₁₆) ou suif (C₁₆ à C₁₈) ou des composés type diols ou cholestérol.

Selon une caractéristique particulière préférée de l'invention, l'alcool gras précité contient de 14 à 22 atomes de carbone et consiste de préférence en un mélange d'alcools ayant de 16 à 18 atomes de carbone provenant du suif ou du coprah.

Il est encore à noter que les compositions auto-émulsionnables utilisées ou réalisées conformément à l'invention se présentent généralement, après filtration des produits provenant de l'éventuelle polycondensation des oses, sous forme d'une cire solide, pâteuse ou liquide selon la nature de l'alcool gras utilisé.

Des exemples de catalyseur acide pouvant être utilisés pour la préparation des compositions auto-émulsionnables précitées sont les acides sulfurique, phosphorique, chlorhydrique, hypophosphoreux et leurs mélanges.

La quantité de catalyseur acide utilisée sera d'environ 0,001 à 0,05 mole par mole de monomère d'ose.

La quantité de catalyseur peut être utilisée pour contrôler la vitesse de la réaction.

Par ailleurs, la température réactionnelle sera généralement comprise entre environ 90 et environ 120°C, et la durée de la réaction entre environ 3 et environ 6 h, de préférence entre 4 et 5 h.

D'une façon générale, ces conditions réactionnelles, ainsi que l'excès d'alcool gras, pourront être facilement déterminées par l'homme de métier connaissant les poids moléculaires de l'alcool gras et de l'ose utilisés, ainsi que la quantité d'alcool gras non éthérifié à obtenir en fin de réaction.

La nature de l'alcool gras et de l'ose sera telle que définie précédemment.

Les compositions auto-émulsionables précitées peuvent par exemple être utilisées pour la préparation de laits ou crèmes, notamment à base d'huile ou cires végétales, d'huile polaire, d'huile minérale et d'huile de silicone.

A cet égard, il a été constaté, de façon tout à fait inattendue, que les compositions auto-émulsionnables utilisées selon l'invention sont supérieures, au niveau de leur pouvoir émulsionnant, aux produitsémulsionnantsclassiques, notamment vis-à-vis des huiles végétales ou des huiles de silicone qui sont très difficiles à émulsionner.

L'invention sera illustrée plus en détail par les exemples suivants, donnés uniquement à titre illustratif.

Dans ces exemples, les pourcentages sont exprimés en poids, sauf indication contraire.

### Exemple 1

### Préparation d'une composition autoémulsionnable

On fait réagir 3 moles d'alcool de suif (C₁₆-C₁₈) et 1 mole de glucose anhydre, en présence d'un système catalytique constitué par de l'acide sulfurique (1,5 g/kg) et de l'acide hypophosphoreux à 50 % (2 g/kg). La réaction est conduite sous vide, à une température de 105 ° C environ, pendant 4 à 5 h.

Après neutralisation à la soude, le produit réactionnel est filtré ou essoré afin d'éliminer les polycondensats de glucose.

Lorsque la réaction est complète (durée suffisante), il n'y a pratiquement aucun polycondensat de glucose, de sorte qu'une telle neutralisation n'est pas nécessaire.

Le produit obtenu par la mise en oeuvre de ce procédé présente un point de fusion d'environ 45 ° C et son indice d'acide est inférieur à 1.

Ce produit à la composition suivante :

| | |
|---|---|
| Alcool cétylstéarylique (C16-C18) | 87,2 % |
| Cétéarylglucosides | 12,2 % |
| Glucose | 0,6% |

On constate que ce produit s'émulsionne facilement à chaud (60-70 ° C) dans l'eau pour donner, selon les quantités mises en oeuvre, soit une crème, soit un lait.

A titre illustratif, on a fabriqué une crème et un lait en mélangeant les proportions indiquées ci-dessous.

### Crème :

| | |
|---|---|
| composition auto-émulsionnable de l'exemple 1 | 25 % |
| eau | 75 % |

Cette crème présente une viscosité d'environ 25 000 mPa·s (cps) à 20 ° C.

### Lait:

| | |
|---|---|
| composition autoémulsionnable de l'exemple 1 | 5 % |
| eau | 95 % |

Ce lait à une viscosité d'environ 3 200 mPa·s (cps) à 20 ° C.

Il est à noter que la composition auto-émulsionnable de l'exemple 1 se présente sous forme d'une cire et que la tolérance oculaire et cutanée de cette cire est parfaite (les indices primaires cutanés et d'irration oculaire étant nuls).

Cette composition auto-émulsionnable est également dépourvue d'impuretés toxiques telles que 1-4-dioxanne, oxyde d'éthylène libre ou amines pouvant former des nitrosamines.

### Exemple 2

### Préparation d'une composition auto-émulsionnable conforme à l'invention

Une cire auto-émulsionnable possédant des propriétés similaires à celles de l'exemple 1 est obtenue en remplaçant l'alcool de suif par de l'alcool oléique (oléine bidistillée).

Le produit obtenu présente la composition suivante :

| | |
|---|---|
| Alcool oléique | 81,3 % |
| Oleylglucosides | 18 % |
| Polydextrose | 0,7 % |

### Exemple 3

### Exemples d'émulsions pouvant être obtenues à partir des compositions auto-émusionnables selon les exemples 1 et 2

### 1/ Crème hydratante peaux grasses :

| | |
|---|---|
| Composition auto-émulsionnable l'exemple 1 (Cire I) | 5 % (p/p) |
| Cétylstéaryloctanoate | 8 % |
| Octylpalmitate | 2 % |
| Polyacrylamide (SEPIGEL 305) | 0,6 % |
| Mucopolysaccharides (SOLABIA) | 5,0 % |
| MICROPEARL M 100 | 3,0 % |
| Conservateur (SEPICIDE HB) | 0,8 % |
| Parfum (Boretta PN 2305 Quest) | 0,2 % |
| Eau distillée | qsp 100 % |

Viscosité obtenue environ 22 000 mPa·s (mpa) à 25 °C.

L'émulsification est obtenue par simple fusion de la Cire I et la phase grasse, qui est mélangée sous agitation mécanique lente avec la phase aqueuse préchauffée à 60°C.

Cette crème est stable à 25°C et 40 ° C plusieurs mois.

### 2/ Crème capillaire démélante :

| | |
|---|---|
| Cire I | 2,8 % |
| Cétyltriméthylammonium chlorure (CTAC) | 3,0 % |
| Kathon CG (conservateur) | 0,1 % |
| Parfum | 0,2 % |
| Eau déminéralisée | qsp 100 % |

On obtient une crème stable par simple émulsification du cationique démêlant (CTAC) par la Cire I fondue à 60°C et mélangée à l'eau à cette même température.

### 3/ Emulsion démaquillante à l'huile d'amandes douces :

Les huiles végétales sont réputées difficiles à émulsionner.

La Cire I permet l'émulsification aisée de l'huile d'amandes douces avec une excellente stabilité.

| | |
|---|---|
| Cire I | 5 % |
| Huile amandes douces | 5 % |
| Polyacrylamide (SEPIGEL 305) | 0,3 % |
| Glycérine | 5 % |
| Conservateur | 0,2 % |
| Parfum | 0,3 % |
| Eau distillée | qsp 100 % |
| Aspect : crème blanche brillante. | |

### 4/ Crème de nuit :

| | |
|---|---|
| Cire I | 5 % |
| Cétylstéaryloctanoate | 10 % |
| Octylpalmitate | 5 % |
| Huile Silicone (DC 200/350) | 2 % |
| Palmitate Glycol | 1 % |
| Mucopolysaccharides | 5 % |
| Micropearl M100 | 5 % |
| Eau | qsp 100 % |

### 5/ Emulsion fluide (base pommade) :

| | |
|---|---|
| Composition auto-émulsionnable de l'exemple 2 (oléique) | 10 % |
| Cétylstéaryloctanoate | 5 % |
| Conservateur | 0,2 % |
| Eau distillée | qsp 100 % |

### Exemple 4

### A) Préparation d'une composition autoémulsionnable

On fait réagir 80 g d'un mélange d'alcools gras (C12 : 25 % ; C14 : 25 % ; C16 : 25 % ; C18 : 25 %) o avec 20 g de butylglucoside (commercialisé sous marque ORAMIX BG 14 par SEPPIC - FRANCE) à 80°C en présence d'acide sulfurique à pH = 1, durant 3 heures, tout en distillant le butanol formé. La transethérification conduit à la composition réactionnelle suivante :

| | |
|---|---|
| Alcool gras (C12 à C18) | 64,3 % |
| Alkyl (C12 à C18) glucosides | 33,8 % |
| Butylglucoside | 1,9 % |

Le produit obtenu s'émulsionne facilement à 50-60 ° C dans l'eau pour donner sous agitation mécanique une émulsion de consistance variable :

### B) Exemples d'utilisation de la composition autoémulsionnable ainsi préparée

a- Lait fluide et onctueux de viscosité 2800 mPa**·**s (cPs), obtenu en mélangeant :
   7 % produit selon A
   93 % eau déminéralisée
b- Crème épaisse et stable de viscosité 75 000 mPa·s (cPs), obtenue en mélangeant :
   21 % produit selon A
   79 % eau déminéralisée

## Revendications

1. Utilisation de compositions à base d'alcools gras comprenant :
- 60 à 90 % en poids d'au moins un alcool gras ayant de 12 à 22 atomes de carbone, de préférence de 16 à 18 atomes de carbone ;
- 10 à 40 % en poids d'un alkylpolyoside, de préférence un alkypolyoside dont la partie alkyle est identique à celle de l'alcool gras ;
- et éventuellement 0,5 à 5 % de polyoside comme compositions auto-émulsionnables pour la préparation d'émulsions.

2. Utilisation selon la revendication 1, **caractérisée en ce que** l'alkylpolyoside précité comprend au moins un ose choisi parmi le groupe constitué de glucose ou dextrose, saccharose, fructose, galactose, maltose, maltotriose, lactose, cellobiose, mannose, ribose, dextrane, talose, allose, xylose, lévoglucosane, cellulose et amidon : de préférence, glucose, dextrose, fructose et maltose.

3. Utilisation selon la revendication 1 ou 2, **caractérisée en ce que** l'alcool gras précité présente de 14 à 22 atomes de carbone et consiste de préférence en un mélange d'alcool ayant de 16 à 18 atomes de carbone, provenant du suif ou du coprah.

4. Utilisation selon l'une des revendications 1 à 3 pour la préparation de laits ou crèmes, notamment à base d'huile végétale, d'huile polaire et d'huile de silicone.

5. Procédé de préparation d'émulsions, **caractérisé en ce qu'**il comprend :
a) la préparation d'une composition auto-émulsionnable comprenant :
- 60 à 90 % en poids d'au moins un alcool gras ayant de 12 à 22 atomes de carbone, de préférence de 16 à 18 atomes de carbone ;
- 10 à 40 % en poids d'un alkylpolyoside, de préférence un alkylpolyoside dont la partie alkyle est identique à celle de l'alcool gras ;
- et éventuellement, 0,5 à 5 % de polyoside ; et
b) la dispersion à chaud de ladite composition, par exemple entre environ 50°C et environ 80°C, dans de l'eau ou un milieu polaire approprié, par simple agitation, notamment mécanique, lente.

6. Procédé selon la revendication 5, **caractérisé en ce que** la composition auto-émulsionnable précitée est obtenue directement par réaction, en milieu acide entre au moins un alcool gras ayant de 12 à 22 atomes de carbone, de préférence de 16 à 18 atomes de carbone, et au moins un ose, dans des conditions réactionnelles, notamment en excès d'alcools gras, telles que le produit réactionnel comprerme 60 à 90 % en poids d'au oins un alcool gras ayant de 12 à 22 atomes de carbone, de préférence de 16 à 18 atomes de carbone ; 10 à 40 % en poids d'un alkylpolyoside, de préférence un alkylpolyoside dont la partie alkyle est identique à celle de l'alcool gras ; et éventuellement 0,5 à 5% de polyoside ; suivie éventuellement d'une neutralisation et d'une filtration.

7. Procédé selon la revendication 5, **caractérisé en ce que** la composition auto-émulsionnable précitée est obtenue par simple mélange de :
- 60 à 90% en poids d'au moins un alcool gras ayant de 12 à 22 atomes de carbone, de préférence de 16 à 18 atomes de carbone ;
- 10 à 40 % en poids d'un alkylpolyoside, de préférence un alkylpolyoside dont la partie alkyle est identique à celle de l'alcool gras ;
- et éventuellement 0,5 à 5 % de polyoside.

8. Procédé selon l'une des revendications 5 à 7, **caractérisé en ce que** l'alkylpolyoside utilisé à l'étape a) pour la préparation de la composition auto-émulsionnable précitée comprend au moins un ose choisi parmi le groupe constitué de glucose ou dextrose, saccharose, fructose, galactose, maltose, maltotriose, lactose, cellobiose, mannose, ribose, dextrane, talose, allose, xylose, lévoglucosane, cellulose et amidon : de préférence, glucose, dextrose, fructose et maltose.

9. Procédé selon l'une des revendications 5 à 8, **caractérisé en ce que** l'alcool gras utilisé à l'étape a) pour la préparation de la composition auto-émulsionnable précitée présente de 14 à 22 atomes de carbone et consiste de préférence en un mélange d'alcools ayant de 16 à 18 atomes de carbone, provenant du suif ou du coprah.

## Patentansprüche

1. Verwendung von Zusammensetzungen auf Basis von Fettalkoholen, umfassend:
- 60 bis 90 Masse-% zumindest eines Fettalkohols mit 12 bis 22 Kohlenstoffatomen, vorzugsweise 16 bis 18 Kohlenstoffatomen;
- 10 bis 40 Masse-% eines Alkylpolysaccharids, vorzugsweise eines Alkylpolysaccharids, dessen Alkyl-Gruppe mit jener des Fettalkohols identisch ist;
- und gegebenenfalls 0,5 bis 5 % Polysaccharid; als auto-emulgierbare Zusammensetzungen zur Herstellung von Emulsionen.

2. Verwendung nach Anspruch 1, **dadurch gekennzeichnet, daß** das Alkylpolysaccharid zumindest eine Ose, ausgewählt aus der Gruppe bestehend aus Glucose oder Dextrose, Saccharose, Fructose, Galactose, Maltose, Maltotriose, Lactose, Cellobiose, Mannose, Ribose, Dextran, Talose, Allose, Xylose, Levoglucosan, Cellulose und Amidon; vorzugsweise Glucose, Dextrose, Fructose und Maltose, umfaßt.

3. Verwendung nach Anspruch 1 oder 2, **dadurch gekennzeichnet, daß** der Fettalkohol 14 bis 22 Kohlenstoffatome aufweist, und vorzugsweise aus einer Mischung von Alkoholen mit 16 bis 18 Kohlenstoffatomen, die von Talg oder Kopra stammen, besteht.

4. Verwendung nach einem der Ansprüche 1 bis 3 zur Herstellung von Milchen oder Cremen, insbesondere auf Basis von Pflanzenölen, polaren Ölen und Silikonölen.

5. Verfahren zur Herstellung von Emulsionen, **dadurch gekennzeichnet, daß** es umfaßt:
a) die Herstellung einer auto-emulgierbaren Zusammensetzung, umfassend:
- 60 bis 90 Masse-% zumindest eines Fettalkohols mit 12 bis 22 Kohlenstoffatomen, vorzugsweise 16 bis 18 Kohlenstoffatomen;
- 10 bis 40 Masse-% eines Alkylpolysaccharids, vorzugsweise eines Alkylpolysaccharids, dessen Alkyl-Gruppe mit jener des Fettalkohols identisch ist;
- und gegebenenfalls 0,5 bis 5 Polysaccharid; und
b) die heiße Dispersion der Zusammensetzung, beispielsweise zwischen ungefähr 50°C und ungefähr 80°C, in Wasser oder einem geeigneten polaren Medium durch einfaches, insbesondere mechanisches, langsames Rühren.

6. Verfahren nach Anspruch 5, **dadurch gekennzeichnet, daß** die auto-emulgierbare Zusammensetzung direkt erhalten wird durch die Reaktion, in einem sauren Medium, zwischen zumindest einem Fettalkohol mit 12 bis 22 Kohlenstoffatomen, vorzugsweise 16 bis 18 Kohlenstoffatomen, und zumindest einer Ose, unter Reaktionsbedingungen, insbesondere in einem Fettalkohol-Überschuß, so daß das Reaktionsprodukt 60 bis 90 Masse-% zumindest eines Fettalkohols mit 12 bis 22 Kohlenstoffatomen, vorzugsweise 16 bis 18 Kohlenstoffatomen; 10 bis 40 Masse-% eines Alkylpolysaccharids, vorzugsweise eines Alkylpolysaccharids, dessen Alkyl-Gruppe mit jener des Fettalkohols identisch ist; und gegebenenfalls 0,5 bis 5 % Polysaccharid umfaßt; gegebenenfalls gefolgt von einer Neutralisation und einer Filtration.

7. Verfahren nach Anspruch 5, **dadurch gekennzeichnet, daß** die auto-emulgierbare Zusammensetzung erhalten wird durch das einfache Mischen von
- 60 bis 90 Masse-% zumindest eines Fettalkohols mit 12 bis 22 Kohlenstoffatomen, vorzugsweise 16 bis 18 Kohlenstoffatomen;
- 10 bis 40 Masse-% eines Alkylpolysaccharids, vorzugsweise eines Alkylpolysaccharids, dessen Alkyl-Gruppe mit jener des Fettalkohols identisch ist;
- und gegebenenfalls 0,5 bis 5 % Polysaccharid.

8. Verfahren nach einem der Ansprüche 5 bis 7, **dadurch gekennzeichnet, daß** das in Schritt a) zur Herstellung der auto-emulgierbaren Zusammensetzung verwendete Alkylpolysaccharid zumindest eine Ose, ausgewählt aus der Gruppe bestehend aus Glucose oder Dextrose, Saccharose, Fructose, Galactose, Maltose, Maltotriose, Lactose, Cellobiose, Mannose, Ribose, Dextran, Talose, Allose, Xylose, Levoglucosan, Cellulose und Amidon; vorzugsweise Glucose, Dextrose, Fructose und Maltose, umfaßt.

9. Verfahren nach einem der Ansprüche 5 bis 8, **dadurch gekennzeichnet, daß** der in Schritt a) zur Herstellung der auto-emulgierbaren Zusammensetzung verwendete Fettalkohol 14 bis 22 Kohlenstoffatome aufweist, und vorzugsweise aus einer Mischung von Alkoholen mit 16 bis 18 Kohlenstoffatomen, die von Talg oder Kopra stammen, besteht.

## Claims

1. Use of fatty alcohol based compositions comprising:
- 60 to 90% by weight of at least one fatty alcohol having from 12 to 22 carbon atoms, preferably from 16 to 18 carbon atoms,
- 10 to 40% by weight of an alkylpolyoside, preferably an alkylpolyoside of which the alkyl part is identical to that of the fatty alcohol, and
- if appropriate, 0.5 to 5% of polyoside, as self-emulsifiable compositions for preparing emulsions.

2. Use according to claim 1, **characterized in that** said alkylpolyoside comprises at least one ose selected from the group constituted of glucose or dextrose, saccharose, fructose, galactose, maltose, maltotriose, lactose, cellobiose, mannose, ribose, dextrane, talose, allose, xylose, levoglucosane, cellulose and starch; preferably glucose, dextrose, fructose and maltose.

3. Use according to claim 1 or 2, **characterized in that** said fatty alcohol has from 14 to 22 carbon atoms and preferably consist in a mixture of alcohol having from 16 to 18 carbon atoms, originating from tallow or copra.

4. Use according to one of claims 1 to 3 for preparing milks or creams, notably based on vegetable oil, polar oil and silicon oil.

5. Method of preparing emulsions, **characterized in that** it comprises the steps of:
a) preparing a self-emulsifiable composition comprising:
- 60 to 90% by weight of at least one fatty alcohol having from 12 to 22 carbon atoms, preferably from 16 to 18 carbon atoms,
- 10 to 40% by weight of an alkylpolyoside, preferably an alkylpolyoside of which the alkyl part is identical to that of the fatty alcohol, and
- if appropriate, 0.5 to 5% of polyoside; and
b) hot-dispersing said composition, for example between about 50°C and about 80°C, in water or in a suitable polar medium, by simple, slow, notably mechanical, stirring.

6. Method according to claim 5, **characterized in that** said self-emulsifiable composition is directly obtained by reaction, in acid medium, of at least one fatty alcohol having from 12 to 22 carbon atoms, preferably 16 to 18 carbon atoms, with at least one ose, in reaction conditions, notably with an excess of fatty alcohols, such that the reaction product contains 60 to 90% by weight of at least one fatty alcohol having from 12 to 22 carbon atoms, preferably 16 to 18 carbon atoms; 10 to 40% by weight of an alkylpolyoside, preferably an alkylpolyoside of which the alkyl part is identical to that of the fatty alcohol; and if appropriate 0.5 to 5% of polyoside; followed, if appropriate, by neutralization and filtration.

7. Method according to claim 5, **characterized in that** said self-emulsifiable composition is obtained simply by mixing:
- 60 to 90% by weight of at least one fatty alcohol having from 12 to 22 carbon atoms, preferably from 16 to 18 carbon atoms,
- 10 to 40% by weight of an alkylpolyoside, preferably an alkylpolyoside of which the alkyl part is identical to that of the fatty alcohol, and
- if appropriate, 0.5 to 5% of polyoside.

8. Method according to one of claims 5 to 7, **characterized in that** the alkylpolyoside used in step a) for preparing said self-emulsifiable composition comprises at least one ose selected from the group constituted of glucose or dextrose, saccharose, fructose, galactose, maltose, maltotriose, lactose, cellobiose, mannose, ribose, dextrane, talose, allose, xylose, levoglucosane, cellulose and starch; preferably glucose, dextrose, fructose and maltose.

9. Method according to one of claims 5 to 8, **characterized in that** the fatty alcohol used in step a) for preparing said self-emulsifiable composition has from 14 to 22 carbon atoms and preferably consists of a mixture of alcohols having 16 to 18 carbon atoms, originating from tallow or copra.
